# EUROPEAN PATENT APPLICATION

(11) **EP 2 842 564 A1**
(43) Date of publication of application: **04.03.2015**
(21) Application number: 13004479.5
(22) Date of filing: 13.09.2013
(51) Int. Cl.: A61K 36/185, B01D 11/00

(54) **Method for the chemical processing of outer birch bark and apparatus for its realisation**

(30) Priority: 30.08.2013 LV 130123
(71) Applicant: "Latvian State Institute of Wood Chemistry" Derived public person, 1006 Riga (LV)
(72) Inventor: Zandersons, Janis, LV-1069 Riga (LV); Rizhikovs, Janis, LV-1013 Riga (LV); Pazhe, Aigars, LV-1006 Riga (LV); Dobele, Galina, LV-2101 Babites district (LV); Tardenaka, Ausma, LV-1083 Riga (LV); Spince, Baiba, LV-1024 Riga (LV); Vilhelmine, Jurkjane, LV-1082 Riga (LV)
(74) Representative: Dolgicere, Nina

(57) **Abstract**

The invention relates to methods and apparatus, designed for the chemical processing of outer birch bark for obtaining triterpene compounds - betulin and lupeol as well as suberinic acids, used in the chemical and pharmaceutical, food and cosmetic industries.

The technical task to be solved with this invention is to develop a simple and efficient method for the chemical processing of outer birch bark for obtaining betulin, lupeol and suberinic acid salts in one apparatus and one outer birch bark processing cycle. According to the present invention, the advanced technical task is attained by a method for chemical processing of outer birch bark, wherein the betulin from the previously ground outer birch bark is isolated with organic solvents in a closed extraction technological complex with the solvent's recovery and the return of the condensate in the recycle, the extract flow is passed through a crystalliser, and then the extract is passed to an evaporator, a heater and a condenser, and the condensed Solution is introduced to the extractor again. In the crystalliser, the crystalline betulin is settled, but lupeol is isolated by crystallisation from the precipitate in the evaporator. After accomplishing the extraction process, the outer birch bark in the extractor is depolymerised, using KOH.

The proposed method and apparatus enabled realising the complex obtaining of betulin and lupeol, as well as suberin derivatives in the continuous process with the solvent recycle, wherein the yield of betulin and lupeol is enhanced, in comparison with the known methods. Besides, this method made it possible to shorten many times the extraction time, simultaneously enhancing the efficiency

## Description

### Field of the invention

The invention relates to methods and apparatus, designed for the chemical processing of outer birch bark for obtaining triterpene compounds - betulin and lupeol as well as suberinic acids, used in the chemical and pharmaceutical, food and cosmetic industries.

### Background of the invention

The conventional method for isolating betulin from outer birch bark is the extraction with organic solvents. As solvents, aliphatic carbohydrates and their chlorine derivatives (dichloroethane, chloroform, carbon tetrachloride, trichloroethylene and perchlorethylene), C₁-C₄ alcohols, ethers, and acetone are used. There is known a method for betulin extraction with hexane (RU 2206572C1), high boiling carbohydrates (RU 2138508C1), toluene (RU 2192879C1), petroleum ether and toluene mixture (RU 2184120C1), ethyl alcohol (RU 2172178C1) and butyl alcohol (RU 2234936C1).

The conventional method for isolating suberinic acids from outer birch bark is its processing with alkaline water - alcohol solution.

There is known a method for chemical processing of outer birch bark, with simultaneous obtaining of betulin and suberinic acid salts, according to which outer birch bark is treated with an alkali water - alcohol solution, filtered and treated with direct steam for distillation of alcohol (SU382657). As a result, a mixture is obtained, which contains betulin, sodium suberinate and lignin, which are then separated from each other. This method is laborious and does not allow obtaining high-quality betulin and sodium suberinate due to the complicated isolation from the obtained mixture. For this reason, the isolation of betulin and suberinic acid salts is carried out gradually, firstly isolating betulin, and then suberinic acid salts.

There is known a method for betulin extraction, with heating the outer birch bark in a reactor with a condenser (RU2184120C1), which allows the return of the condensate into the extractor. The extract's discharge from the still pot is carried out once at the end of the extraction process.

This method is not efficient and is inferior to the classical extraction in the Soxhlet apparatus in terms of both the process duration and the solvent and energy consumption.

There is known also a method, which is carried out in a closed extraction-regeneration set on the basis of the Soxhlet apparatus. The method includes the loading of the ground outer birch bark in an extractor, but the solution - in an evaporation still pot, heating of the solvent till boiling and the continuous feeding of the steam portions from the evaporation still pot to a condenser. The condensed steam is passed to the extractor; after its filling, the extract is discharged into the evaporation still pot, the still pot content is heated till boiling for distilling off the solvent and its use for the further extraction stage (RU2206572).

This method has several drawbacks. Firstly, the method does not ensure the high efficiency of the extractives' solubility, because it envisages repeated preparing of extracts, but the formed flux is laminar, with an immobile boundary layer between the solid and liquid phases, which is an obstacle for achieving a high concentration gradient. Secondly, the extraction process is lasting, since it occurs at a temperature below the boiling point ot the solution.

The second main component of outer birch bark is a polyester, suberin, which makes up 38-40% of the outer birch bark mass. Polyester is formed by higher -C₁₆ - C₂₂ fatty acids, the molecules of which contain hydroxyl groups and epoxide groups, and a part of the acids have two carboxyl groups; therefore, suberin is a substance, insoluble in water and any organic solvent, since polyester forms a spatial structure. To obtain suberinic acid salts or suberinic acids in the free form, hydrolytic depolymerisation of suberin in a basic medium should be carried out, obtaining the corresponding salts, acidifying which, free acids can be isolated.

Basic depolymerisation is commonly carried out in an organic polar solvents' medium, most often using aliphatic alcohols (ethanol, isopropanol, etc.). The main reason is that, in the organic solvents' medium, the suberin depolymerisation is more complete, and more individual acid salts are formed, acidifying which till pH 5-6, a great amount of suberinic acids, containing epoxy acids (mainly 9,10-epoxy-18-hydroxy octadecanoic acid), are obtained. If suberin is depolymerised in the water medium, the yield of individual fatty acids is lower, and the epoxy groups are not retained, but form two hydroxyl groups. The latter variant can be unprofitable, if the acid is disadvantageous to be used in organic synthesis.

Thus, according to the European patent application 2,371.805 A1 (2005. 21. X), in the isopropanol solution, in which sodium hydroxide is dissolved, the ground outer birch bark is boiled for 1 hour and filtered, the solution (filtrate) is boiled for another 15 minutes and, after 24 hour holding in a freezer, the first salt's fraction is filtered. The salts' fractionated crystallisation is repeated twice again. In the first two salts' fraction, 50-60% are 9,10-epoxy-18-hydroxy octadecanoic acid salts. The method is extremely laborious and justifies itself if the aim is the fine organic synthesis on the epoxy acids base.

According to the USA patent 6.768.016 B2 (2004. 27 VII), the pelletised extracted outer birch bark is also saponified with sodium hydroxide isopropanol solution for 1 hour, the solution is discharged, and the outer birch bark is boiled with isopropanol for another 20 minutes. The solutions are evaporated, diluted with water, and the insoluble 9,10-epoxy-18-hydroxy-octadecanoic acid salts (50-70% from the mass) are separated. Acidifying the filtrate till pH 5.5-6.5, a suberinic acid fraction with a high content of 9,10-epoxy-18-hydroxy-octadecanoic acid (50-70% from the mass) is obtained. The method, also in this case, justifies itself, if the aim is individual acids for organic synthesis.

A related method for hydrolytic depolymerisation in basic water medium is recommended for the World Intellectual Property Organization (WIPO) in the patent application WO/2010/093220 A (2010. 10.11). In the patent, improved contacting of suberin with alkali is designed, using outer birch bark vacuum treatment prior to the alkali solution's pouring to the outer birch bark. Boiling under reflux for 1 hour and filtering are carried out. The filtrate is acidified till pH above 6 and left in the cold overnight. 9,10-epoxy-18-hydroxy-octadecanoic acid with the purity of 72% settles. The advantage of the method is the water medium, but the non-indicated yield makes to doubt whether it would be high.

Among the known methods, most related to the claimed method is a method for the chemical processing of outer birch bark, which includes the isolation of betulin from ground outer birch bark, extracting with an organic solvent flow in the closed extraction technological complex. In this case, extraction is carried out in a flowing through type extractor at the solvent's boiling temperature, with continuous feeding of the extract's portions to an evaporation still pot, wherein the solvent's recovery from the extractor and the evaporation still pot in a condenser, the solvent's steam condensation, and the condensate's return to the extractor, then purification of betulin and obtaining of sodium suberinate from the extracted outer birch bark (RU2306318C2) are realised.

This method is complicated because the extraction is carried out at the solvent's boiling temperature. The drawbacks of the known method include also the choice of the solvent aliphatic alcohol (ethanol, isopropanol), which makes it possible to increase the yield of the extracted substances, but, at the same time, to complicate considerably the isolation of betulin, lupeol and other triterpenes in the pure form. Besides, the use of toluene as a betulin crystallisation medium does not enable the use of the isolated betulin in the pharmaceutical industry.

The technical task to be solved with this invention is to develop a simple and efficient method for the chemical processing of outer birch bark for obtaining betulin, lupeol and suberinic acid salts in one apparatus and one outer birch bark processing cycle.

### Summary of the invention

According to the present invention, the advanced technical task is attained by a method for chemical processing of outer birch bark, wherein the betulin from the previously ground outer birch bark is isolated with organic solvents in a closed extraction technological complex with the solvent's recovery and the return of the condensate in the recycle, the extract flow is passed through a crystalliser, and then the extract is passed to an evaporator, a heater and a condenser, and the condensed solution is introduced to the extractor again. In the crystalliser, the crystalline betulin is settled, but lupeol is isolated by crystallisation from the precipitate in the evaporator. After accomplishing the extraction process, the outer birch bark in the extractor is depolymerised, using KOH.

The apparatus for realising the method consists of a flowing through type extractor, an evaporator, a heater and a condenser for feeding the recovered solvent in the extractor; in addition, it contains a crystalliser that is connected to the extractor and an additional heater, adjusted between the crystalliser and the evaporator.

### Brief description of the Drawings

The apparatus for chemical processing of outer birch bark is shown in the flow diagram.

The apparatus includes a flow type extractor 1, connected to an intermediate crystalliser 2, which is connected to an evaporator 4 with the help of an intermediate crystalliser mother liquor heater with a steam jacket 3. The evaporator 4 is connected to a condenser 6 with the help of a solvent vapour reduction pipe with a heating jacket 5.

In the extractor 1 lid, a mechanical mixer 7 with an electrical drive 8 is installed, but inside the extractor 1, a wire net basket 9, covered with a filter material, is installed, in which the ground outer birch bark is poured in. The extractor 1 has a steam jacket. The extractor 1 is connected with the atmosphere through a reflux cooler 10.

### Detailed Description of the Invention

Before starting the work, the outer birch bark is ground, and pellets are formed, which are then crashed to destroy the cell structure and make the raw material more suitable for extraction.

The treated raw material is poured into the basket 9 and placed in the extractor 1, which is then filled with a solvent. The continuous feeding of the fresh solvent in the extractor 1 is ensured by the evaporator 4, which evaporates the mother liquor from the intermediate crystalliser 2. In the intermediate crystalliser 2, the hot extract is continuously discharged from the extractor 1, and in the intermediate crystalliser 2, the extract's temperature falls dramatically, and betulin crystals settle from the extractor. The mother liquor gradually flows to the evaporator 4, where it warms up and evaporates. The mother liquor descent pipe 3 is heated by a steam jacket to prevent the formation of crystals and warm up the mother liquor.

The continuous solvent flow gets into the extractor through the condensation system, the main stage of which is by a steam jacket heated solvent vapour pipe 5 to prevent the condensate's formation and reflux, but in the condenser, the supply of the cooling water is regulated for maintaining the maximally high possible temperature of the condensate. This decreases the heat consumption for maintaining the extractive's temperature in the extractor 1.

In the removable lid of the extractor 1, a mixer 7 with a driver 8 is installed. The mixer 7 is propeller type or turbine type and provides the intensive turbulent flow of the mixture of the extract and the fine-dispersed extracted material in the extractor. Therefore, the desired size of the milled pellets is 0.4-2.0 mm, which ensures an active mass exchange in the system: solvent - solid. Because a known degradation of the solid phase occurs during the process, the solution, which leaves the extractor, is continuously filtered through a filter material, with which the steel wire net sieve 9 is covered for placing and transport of the material within the shop.

As the process is accomplished, the extractor 1 is discharged, supplying the extract to crystallisation and then to evaporation or, after the settled crystals' filtering, used for the extraction of the next portion of pellets, because the summary amount of the extractives in this solution does not exceed 1-2% from the extracted outer birch bark mass. The extracted material is washed with fresh solvent. The washing solution is used for the extraction of the next batch. The extracted outer birch bark is taken out from the extractor, and the solvent is recovered in the drier. The extracted outer bitch bark is used for obtaining suberinic acids or as a binder for pelletising deciduous wood chips or producing fibre boards. In the evaporator, extractives after the crystallisation of the main mass of betulin in the intermediate crystalliser 2, which enter therein with the mother liquor, are accumulated. These extractives contain the left over betulin and an increased concentration of lupeol.

Examples of the realisation of the method for the chemical processing of outer birch bark are given below.

### Example 1

In the stainless steel cylindrical extractor with the geometrical volume 3.5 L (inner diameter 139 mm, height 230 mm), a cylindrical steel wire net with a geometrical volume of 2.65 L (inner diameter 130 mm; height 200 mm) is placed, in which 200 g (in terms of the oven dry mass) of pelletised crashed outer birch bark is fed. The extractor is covered with a lid, in which a mixer and its driver are installed. A reflux and discharge cooler is connected. The extractor is filled till the overflow with 2.3 L of petroleum ether, heated till 100°C, with a boiling temperature of 100-140°C. An intermediate crystalliser (geometrical volume 650 mL) is attached to the extractor, in which 500 mL of the solvent is filled, and the cooling jacket is connected for water supply. The cooling water temperature is maintained no higher than 10°C. In the evaporator, 2 L of the petroleum ether with a boiling temperature of 100-140°C is filled, and with a heated vapour pipe and a condenser, is connected to the extractor. The evaporation intensity is regulated so that the vapour supply rate would be 2 L of the condensate per hour. Thereby, the extract is gradually replaced from the extractor, in which the turbulent regime and a temperature of 103-106°C are permanently maintained. The extract is continuously discharged from the extractor to the intermediate crystalliser through the removable container's sieve and filter material. In the intermediate crystalliser, with decreasing extract's temperature from 106°C to 15-20°C, in 7 hours, 25.8 g of triterpene extractives in the white crystal form, with an average content of betulin and lupeol of 94.6% and 3.1%, respectively, settle.

At the end of the process, cooling the solution collected in the evaporator till 15-20°C, 19.7 g of yellowish crystals is obtained, from the mass of which 73.0% is betulin and 10.6% lupeol. Evaporating the remaining mother liquor till a dry mass, 3.1 g of a yellowish substance is isolated, which contains 7.9% of betulin and 38.9% of lupeol. The solution remaining in the extractor (2.3 L) is discharged through a valve installed at the bottom of the extractor and, for emptying, with the help of a small air overpressure, about 90% of this solution is collected, in which 2.7 g of yellowish crystals is present, wherein 73.8% is betulin and 7.6% lupeol.

The total yield of extractives after the 7 hour intensive mass exchange extraction is 51.3 g or 25.65% from the oven dry outer birch bark mass. For comparison purposes, from the same outer birch bark material, extracting with petroleum ether (boiling temperature 100-140°C) for 33 hours in a Soxhlet apparatus, 17.4% of extractives in terms of the oven dry outer birch bark mass was obtained, with the content of betulin and lupeol of 86.5% and 13.2%, respectively.

### Example 2

As described in Example 1, the extraction process is realised with 200 g (oven dry mass) of crashed outer birch bark pellets, but, as an extractive, 2.3 L of cyclohexane (boiling temperature 80.7°C, d = 0.779 g/cm³) is used, heated up to 78-79°C. As described in Example 1, in the intermediate crystalliser, 500 mL of cyclohexane is filled, which is cooled with a water jacket. The steam supply for ensuring the solution's circulation is maintained by 2 L of cyclohexane, filled in the evaporator. The condensate feeding rate is about 2 L/h.

As the extract flows through the intermediate crystalliser, in 7 hours, 22.8 g of the triterpene mixture in the form of white crystals is settled, with the main components: 90.8% of betulin and 5.02% of lupeol from the oven dry outer birch bark. After 7 hours, as the extraction is accomplished, from the cooling of the solution, collected in the evaporator, 20.1 g of yellowish crystals is collected, from which 75.1% is betulin, but 7.0 % lupeol. In the remaining mother liquor solid, 4.7 g of the main components are betulin and lupeol, namely, 8.75% and 27.0%, respectively. The solution discharged from the extractor contains 2.3 g of yellowish substance with the content of betulin and lupeol of 78.8% and 11.2%, respectively.

The total extractives' yield after the 7 hour intensive mass exchange extraction is 49.9 g or 24.9% from the oven dry outer birch bark mass. For comparison, from the same outer birch bark material, extracting with cyclohexane in a Soxhlet apparatus for 33 hours, 28.4% of extractives was obtained in terms of the oven dry outer birch bark mass, with contents of betulin and lupeol of 92% and 7.5%, respectively.

### Example 3 (with obtaining potassium suberinate)

As described in Example 1, the extraction process is realised in a reactor with a wire net, in which 200 g of crashed outer birch bark pellets is filled and petroleum ether (boiling temperature 100-140°C) is used as an extractive. As described in Example 1, in the intermediate crystalliser, which is cooled with a water jacket, 500 mL of petroleum ether is filled. In the evaporator, 2 L of petroleum ether is filled so that to ensure the condensate's feeding to the extractor - 2 L/hour.

During the 7 hour extraction, flowing through the intermediate crystalliser, 30 g of triterpene crystals (betulin content 93.7%) is gathered.

From the petroleum ether collected in the evaporator, after 7 h, 20.4 g of yellowish crystals (betulin content 74.1%; lupeol content 11.3%) is evaporated. Evaporating the mother liquor, 3.3 g of yellowish substance (containing 8.1% of betulin and 37.9% of lupeol) is obtained.

The total yield of extractives is 53.8 g or 26.9% from the oven dry outer birch bark. From the control sample in the Soxhlet apparatus, within 33 hours, 18.5% of the extract from the oven dry outer birch bark mass is obtained. After the extraction from the extractor with the help of a small (100-150 mm H₂O) air overpressure, the petroleum ether extract is discharged from the lower valve, which is used for the next extraction.

Continuing the heating, a small amount of superheated steam (105-110°C) is filled in the extractor through the lower valve and, using the intermediate crystalliser as a cooler, petroleum ether residue (30 mL) is distilled off from the extracted outer birch bark.

700 mL of the 4.5% potassium hydroxide solution is filled in the extractor and, at 100°C under reflux, is hydrolysed with mixing for 3 hours. The solution is filtered with the help of a small air overpressure (100-150 mm H₂O). 540.0 g of potassium suberinate solution filtrate (dry matter 7.5%; 40.3 g) is collected. The saponified outer birch bark solid residue settlings (365 g; moisture 70.3%; dry matter 108.4 g) caught in the filter are further treated individually or used in a mixture with the filtrate.

Thus, the proposed method and apparatus enabled realising the complex obtaining of betulin and lupeol, as well as suberin derivatives in the continuous process with the solvent recycle, wherein the yield of betulin and lupeol is enhanced, in comparison with the known methods. Besides, this method made it possible to shorten many times the extraction time, simultaneously enhancing the efficiency.

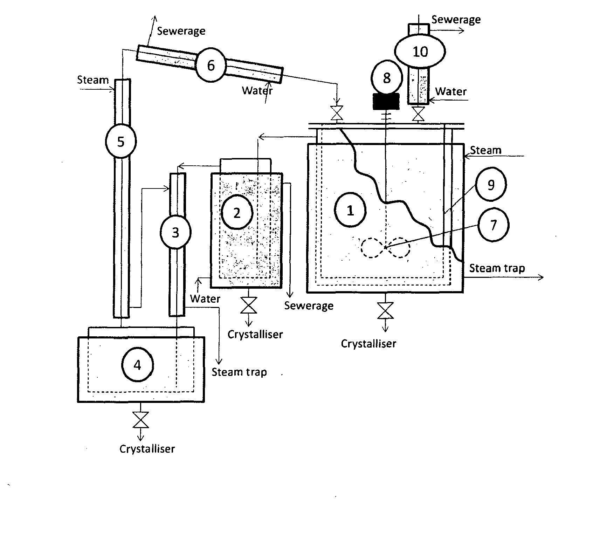

## Claims

1. A method for the chemical processing of outer birch bark, isolating betulin from the preliminarily ground outer birch bark with organic solvents in a closed extraction technological complex with the continuous extract flow, **characterized in that** the extract flow is passed through a crystalliser, while the mother liquor is then heated anew, passed through an evaporator, the vapours through a condenser, and the condensed solvent is introduced in the extractor again.

2. The method according to Claim 1, **characterized in that** betulin is isolated from the extract solution in the intermediate crystalliser.

3. The method according to Claims 1 or 2, **characterized in that** the mother liquor after the recrystallisation of the solution from the evaporator is used for obtaining the lupeol concentrate.

4. An apparatus for the chemical processing of outer birch bark, which includes a flowing through type extractor, connected with an evaporator, which is connected with a crystalliser, a heater and a condenser for feeding the distilled solvent into the extractor, **characterized in that** an additional crystalliser, which is installed between the extractor and the evaporator, is connected to the apparatus.

5. The apparatus according to Claim 4, **characterized in that** a mother liquor heater is installed behind the additional crystalliser.
